(19) Europäisches Patentamt
European Patent Office
Office européen des brevets

(11) Veröffentlichungsnummer: **0 150 466**
**A2**

# (12) EUROPÄISCHE PATENTANMELDUNG

(21) Anmeldenummer: **84116028.6**

(22) Anmeldetag: **21.12.84**

(51) Int. Cl.⁴: **C 07 K 15/00**, G 01 N 33/53, G 01 N 33/569

(30) Priorität: **27.01.84 DE 3402739**

(43) Veröffentlichungstag der Anmeldung: **07.08.85 Patentblatt 85/32**

(84) Benannte Vertragsstaaten: **AT BE CH DE FR GB IT LI LU NL SE**

(71) Anmelder: **BOEHRINGER INGELHEIM DIAGNOSTIKA GMBH, Gutenbergstrasse 3 Postfach 1609, D-8046 Garching bei München (DE)**

(72) Erfinder: **Franken, Norbert, Dr., Wilhelm-Mayr-Strasse 1a, D-8000 München 21 (DE)**
Erfinder: **Kolb, Helmut, Prof. Dr., Kranzhornstrasse 13, D-8000 München 82 (DE)**
Erfinder: **Schleifer, Karl-Heinz, Prof.Dr., Schwalbenstrasse 3a, D-8044 Lohhof (DE)**
Erfinder: **Seidl, Hans Peter, Dr., Marsopstrasse 20, D-8000 München 60 (DE)**
Erfinder: **Tympner, Klaus Dieter, Prof.Dr., Waterloostrasse 70, D-8000 München 71 (DE)**
Erfinder: **Weiss, Ludwig, Prof. Dr., Hauberrisser Strasse 7, D-8000 München 71 (DE)**

## (54) Antikörper gegen den Glycanstrang des Peptidoglycans, Verfahren und Reagenzien zu ihrer Herstellung und Methoden zu ihrer quantitativen Bestimmung.

(57) Die Erfindung betrifft Antikörper aus der Klasse der Immunglobuline G, A und M in biologischem Untersuchungsmaterial gegen den Glycanstrang des Peptidoglycans, die gekennzeichnet sind durch
a) spezifische Bindung an Zuckerderivate der allgemeinen Formel

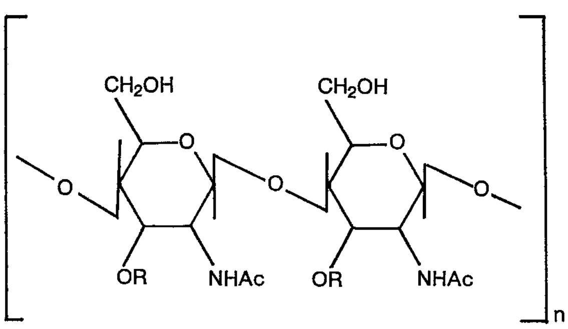

(I)

worin
R Wasserstoff oder einen der Reste

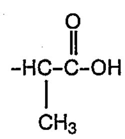

oder

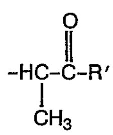

,

R′ den Rest einer Aminosäure oder eines Peptids und
n eine der Zahlen 1 bis 6, vorzugsweise 2–4, bedeuten, oder
b) spezifische Bindung an Zuckerderivate der allgemeinen Formel I enthaltende isolierte bakterielle Zellwände. Sie betrifft ferner ein Verfahren zur Herstellung der Antikörper und zu ihrer quantitativen Bestimmung nach verschiedenen Methoden.

EP 0 150 466 A2

Zum Nachweis von biologisch aktiven Substanzen wie Hormonen, Pharmaka und bestimmten Proteinen, die nur in geringen Konzentrationen in biologischem Untersuchungsmaterial vorkommen, sind hochspezifische und empfindliche Testsysteme nötig. Diese Forderung erfüllen qualitative und quantitative Immunbestimmungen (Immunoassays), die auf der Fähigkeit spezifischer Proteine (Antikörper) beruhen, korrespondierende Stoffe (Antigene und Haptene) zu binden.

Man kennt homogene und heterogene Testsysteme; die Mehrzahl der heute beschriebenen Enzym-Immunoassays verlaufen in heterogener Phase, so z.B. der "Sandwich-Immunoassay" (ELISA), der neben geringer Störanfälligkeit und vielseitiger Anwendbarkeit eine hohe Empfindlichkeit aufweist.

Ein wichtiger Anwendungsbereich des ELISAs besteht in der Messung spezifischer Antikörper für Aussagen des Immunstatus und der Immundiagnose bei Infektionskrankheiten (Carlson, B.A., Giebink, G.S., Spika, J.S. und Gray, E.D. (1982): Measurement of immunoglobulin G and M antibodies to type III pneumococcal polysaccharide by enzyme-linked immuno sorbent assay. J. Clin. Microbiol. 16, 63 - 69)

Insbesondere spielt die Bestimmung von Antikörpern gegen die Zellwandstrukturen von Bakterien eine große Rolle, da in erster Linie diese Moleküle für die Interaktion zwischen pathogenem Mikroorganismus und Wirt verantwortlich sind.
Eine solche Bestimmung besitzt besondere Bedeutung für die Diagnose bakterieller Infektionen, die auf anderen Wegen nicht nachgewiesen werden können.

Die bisher in der Bakteriologie praktizierte Methode des Nachweises einer bakteriellen Infektion über den jeweiligen Erreger (z.B. Staphylokokken, Mycobakterien) ist zeitaufwendig und unsicher.
Daneben ist über herkömmliche Antigen- beziehungsweise Antikörper-Nachweismethoden nur eine erregerspezifische Diagnose möglich. Da jedoch bei einigen Erkrankungen (z.B. Pyelonephritis) verschiedene Erreger in Frage kommen, ist die Diagnose einer bakteriellen Infektion über diese Antigen- beziehungsweise Antikörper-Nachweismethoden nur unter großem Material- und Zeitaufwand möglich.

Der Erfindung lag daher die Aufgabe zugrunde, das allen Bakterien gemeinsame Zellwandantigen, den Glycanstrang des Peptidoglycans, zur Bestimmung spezifischer Immunglobuline G, A und M zu verwenden.

Gegenstand der Erfindung sind somit Antikörper aus der Klasse der Immunglobuline G, A und M in biologischem Untersuchungsmaterial gegen den Glycanstrang des Peptidoglycans, die gekennzeichnet sind durch

a) eine spezifische Bindung an Zuckerderivate der allgemeinen Formel

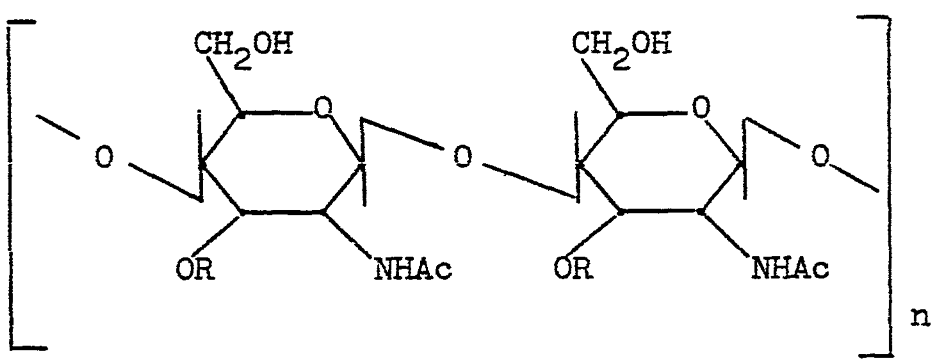

(I)

worin

R Wasserstoff oder einen der Reste

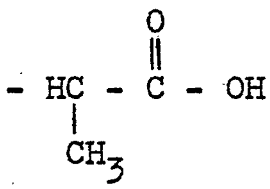

oder

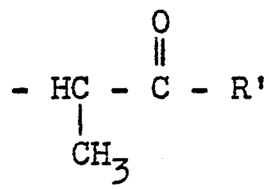

,

R' den Rest einer Aminosäure oder eines Peptids und

n eine der Zahlen 1 bis 6, vorzugsweise 2 - 4 bedeuten, oder

b) eine spezifische Bindung an Zuckerderivate der allgemeinen Formel I enthaltende isolierte bakterielle Zellwände.

Das Makromolekül, von dem ein Ausschnitt aus der Primärstruktur in Formel I wiedergegeben ist, besteht aus unverzweigten Polysaccharidketten von N-Acetyl-Glucosamin und N-Acetyl-Muraminsäure (3-O-D-Milchsäureether des N-Acetyl-Glucosamins), die alternierend ß-1,4 verknüpft sind (Glycanstrang, vgl. nachstehende Abbildung). An die Carboxylgruppe der Muraminsäure schließt sich in der Regel die Peptiduntereinheit der Sequenz L-Ala-D-Glu(L-Diaminosäure-D-Ala-(D-Ala)) an. Diese Peptiduntereinheiten können entweder direkt (z.B. Bacillen) oder über Interpeptidbrücken verknüpft sein (L-$Ala_{2-3}$ bei Streptokokken oder $Gly_5$ bei Staphylokokken). Auf diese Weise entsteht das quervernetzte, hochmolekulare Peptidoglycan, das für die osmotische Stabilität und die Form der Bakterienzelle verantwortlich ist.

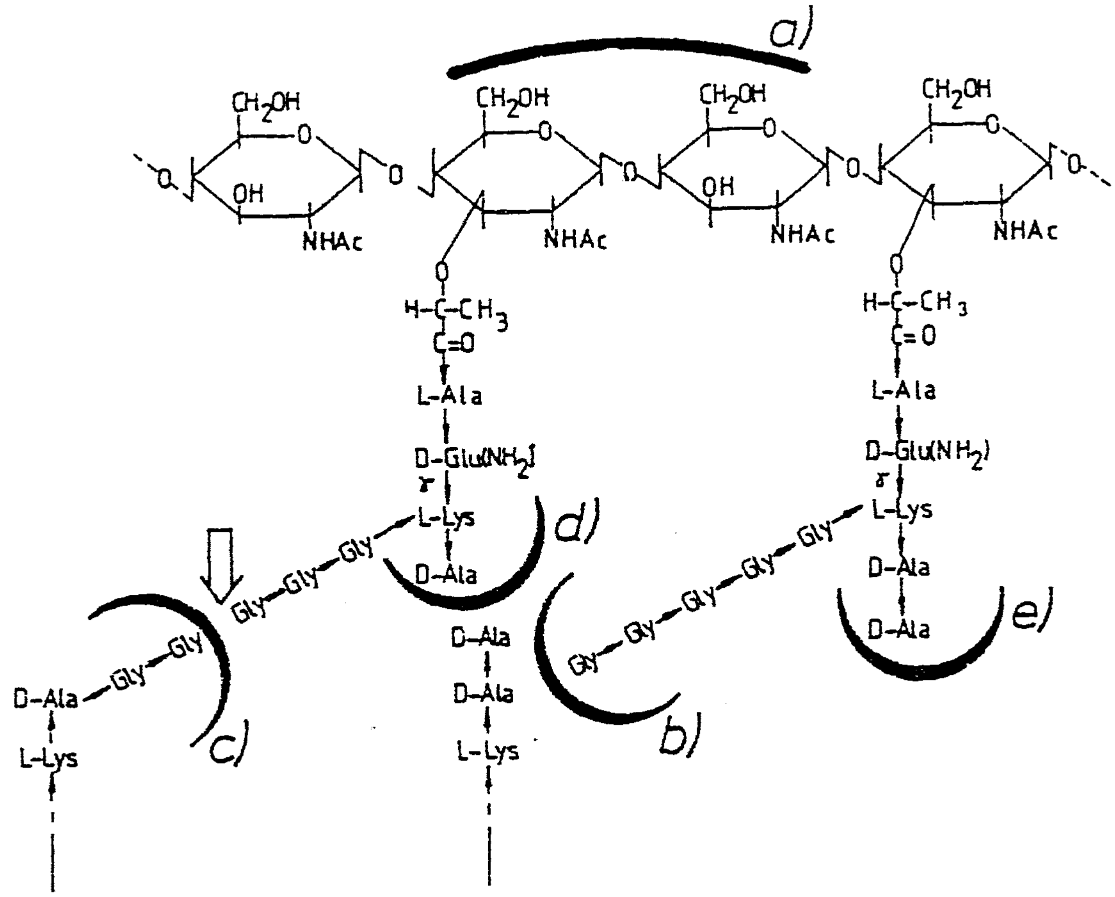

<u>Abbildung:</u> Schematische Darstellung des Peptidoglycans von Staphylococcus aureus und der 5 antigenen Determinanten: Glycanstrang (a), N-Terminus (b) und C-Terminus (c) der Interpeptidbrücke, Tetrapeptid (d) und Pentapeptid (e) der Peptiduntereinheit

Bakterien, die mit dieser Bestimmungsmethode erfaßt werden können, sind z.B. folgende: Staphylokokken, Streptokokken, Bacillen, E. coli, Salmonellen, Shigellen, Pseudomonaden,Neisserien.

Der Peptidoglycan-Glycanstrang, der eine erstaunliche Homogenität aufweist, stellt ein allen Bakterien gemeinsames Antigen dar. Da bei vielen Erkrankungen die verursachende Bakterienart nicht bekannt ist, kann in diesen Fällen nicht nach Antikörpern gefahndet werden, die gegen ein gattungs- beziehungsweise artspezifisches Antigen gerichtet sind. Hier erscheint die Bestimmung von Antikörpern mit Spezifität für ein gemeinsames Antigen aller Bakterien (Glycanstrang des Peptidoglycans) im Hinblick auf eine mögliche diagnostische Bedeutung von besonderem Interesse.

Die vorliegende Erfindung betrifft ferner ein Verfahren zur Herstellung der Antikörper gegen den Glycanstrang des Peptidoglycans sowie Verfahren und Reagenzien zur quantitativen Bestimmung dieser Antikörper in biologischem Untersuchungsmaterial; als solche Verfahren seien genannt der Doppel-Antikörper-Radioimmun-Assay, der Doppel-Antikörper-Enzymimmun-Assay, die nephelometrische Methode, der Festphasen-Sandwich-Radioimmun-Assay, der Festphasen-Enzymimmun-Assay, der Festphasen-Fluoreszenzimmun-Assay sowie der Latex-Agglutinationstest.

Die Bestimmung des Antikörpers wurde wie folgt vorgenommen:

1. Standardgewinnung

Standardlösungen von Antikörpern mit Spezifität gegen den Glycanstrang des Peptidoglycans werden durch Affinitätschromatographie (mit Matrix gebundenen Zuckerderivaten der allgemeinen Formel I) aus Patientenseren, in denen solche Antikörper nachgewiesen wurden, gewonnen.

Nach Bindung der spezifischen Antikörper an die Affinitäts-Matrix wird nichtgebundenes Serumprotein durch Waschen mit Pufferlösungen entfernt. Die Elution der an die feste Matrix gebundenen Antikörper erfolgt durch spezifische Desorption der gebundenen Immunglobuline mit Zuckerderivaten der allgemeinen Formel I. Die Desorption kann ebenfalls durch im Prinzip veröffentlichte Techniken wie Anwendung tiefer pH-Werte oder hoher Salzkonzentrationen erfolgen. Nach Dialyse gegen Pufferlösungen wird der Gehalt an spezifischen Antikörpern gegen den Glycanstrang des Peptidoglycans über den Immunglobulingehalt der gereinigten Fraktion quantifiziert.

2. Testaufbau

Die quantitative Bestimmung von spezifischen Antikörpern der verschiedenen Immunglobulinklassen gegen den Glycanstrang des Peptidoglycans läßt sich mit zwei prinzipiell verschiedenen Methoden durchführen.

a) Quantitative Bestimmung im löslichen System (Messung in homogener Phase)

b) Quantitative Bestimmung durch Messung im heterogenen System (Messung in heterogener Phase; Festphasen-Immuno-Assay).

2.1. Messung in der homogenen Phase, z.B.

2.1.1. quantitative Bestimmung von Antikörpern gegen den Glycanstrang des Peptidoglycans in einem Doppel-Antikörper-Radioimmuno-Assay.

Zuckerderivate der allgemeinen Formel I werden radioaktiv markiert und der Testansatz entsprechend nachfolgendem Schema ausgeführt:

*ZD. + Ak ⟶ *ZD.-Ak

Die Trennung von radioaktivem freien Antigen von radiaktivem Antikörper-gebundenem Antigen erfolgt nach der Doppel-Antikörpermethode durch spezifische Präzipitation der Immunglobuline mit Hilfe eines zweiten Anti-Antikörpers nach folgendem Schema:

*ZD.-Ak + Anti-Ak ⟶ $(*ZD.-Ak)_n$-$(Anti-Ak)_m$-Komplexe

Die radioaktive Markierung des Antigens kann für R' = L-Ala-D-Glu(L-Lys) oder L-Ala-D-Glu($A_2$pm) z.B. erfolgen

a) durch Kopplung von Tyrosin an Zuckerderivate der allgemeinen Formel I und anschließende Jodierung mit $^{125}$J mit konventionellen Methoden,

b) durch Jodierung mit (N-Succinimidyl-3-(4-Hydroxy, 5-($^{125}$J)Jodophenyl)propionat,

c) durch Tritierung des Antigens mit N-Succinimidyl-(2,3-$^3$H)propionat.

Durch selektive Auswahl der Spezifität des zweiten Antikörpers lassen sich separat verschiedene Immunglobulinklassen, wie z.B. IgG oder IgM etc. bestimmen.

Abkürzungen: ZD. = Zuckerderivate der allgemeinen Formel I

$A_2$pm = Diaminopimelinsäure

Es empfiehlt sich, die verwendeten Anti-Antikörper vor Verwendung durch Affinitätschromatographie an Matrix gebundenen Zuckerderivaten der allgemeinen Formel I zur Entfernung von eventuell enthaltenden Antikörpern gegen Peptidoglycan zu reinigen bzw. vorzugsweise monoklonale Anti-Antikörper zu verwenden.

2.1.2. Quantitative Bestimmung von Antikörpern gegen den Glycanstrang des Peptidoglycans in einem Doppel-Antikörper-Enzymimmuno-Assay.

Testaufbau wie unter 2.1.1., jedoch erfolgt die Markierung der Anti-Antikörper mittels eines Enzyms wie z.B. alkalische Phosphatase, Peroxidase, Glucose-Oxidase-Peroxidase, Glucose-6-Phosphat-dehydrogenase, Malat-Dehydrogenase etc. anstelle von Radioaktivität.

2.1.3. Quantitative Bestimmung von Antikörpern gegen den Glycanstrang des Peptidoglycans mittels nephelometrischer Methoden.
Zuckerderivate der allgemeinen Formel I werden an eine lösliche Matrix, z.B. Proteine, Polyvinyl-Pyrrolidon etc. gebunden und der Testansatz entsprechend nachfolgendem Schema ausgeführt:

$$\text{Matrix-(ZD.)}_n + \text{Ak} \longrightarrow \underset{\text{Trübung}}{\text{Matrix-(ZD.)}_n\text{-Ak-Komplexe}}$$

Die Kopplung der Zuckerderivate der allgemeinen Formel I an das Trägermaterial kann z.B. erfolgen
1. durch Bindung der jodacetylierten Zuckerderivate der allgemeinen Formel I an die Matrix
2. mit der Carbodiimid-Methode
3. mittels bifunktioneller Reagenzien, z.B. Diisocyanate, Glutardialdehyd etc..

2.2. Messung in der heterogenen Phase

Zuckerderivate der allgemeinen Formel I werden an eine feste, unlösliche Matrix gebunden, wie z.B. Papier, Kunststoffe, Latex etc.. Die Kopplung erfolgt nach konventionellen Methoden. Die Bindung der Zuckerderivate der allgemeinen Formel I kann entweder direkt an die Matrix erfolgen oder über sogenannte "spacer". Als "spacer" können dienen: aliphatische Kohlenwasserstoffketten, Proteine wie z.B. Albumin, RNase etc..

2.2.1. Quantitative Bestimmung von Antikörpern gegen den Glycanstrang des Peptidoglycans in einem Festphasen-"Sandwich"-Radio-Immuno-Assay unter Verwendung radioaktiv markierter Anti-Antikörper.

2.2.2. Quantitative Bestimmung von Antikörpern gegen den Glycanstrang des Peptidoglycans in einem Festphasen-Enzym-Immuno-Assay.

Testaufbau wie unter 2.2.1., jedoch erfolgt die Markierung der Anti-Antikörper mittels eines Enzyms wie unter 2.1.2. aufgeführt, anstelle von Radioaktivität.

2.2.3. Quantitative Bestimmung von Antikörpern gegen den Glycanstrang des Peptidoglycans in einem Festphasen-Fluoreszenz-Immuno-Assay.

Testaufbau wie unter 2.2.1., jedoch erfolgt die Markierung des Anti-Antikörpers mittels eines Fluoreszenz-Farbstoffes (vgl. auch FIAX®- StiQ™-System).

2.2.4. Quantitative Bestimmung von Antikörpern gegen den Glycanstrang des Peptidoglycans mittels eines Latex-Agglutinationstestes.

Die Zuckerderivate der allgemeinen Formel I werden entweder direkt oder über "spacer", wie z.B. Proteine, an Latexpartikel gekoppelt.

Bei den unter 2.1. sowie unter 2.2. aufgeführten quantitativen Bestimmungsmethoden erfolgt die Quantifizierung mittels Eichkurve, die mit Hilfe der unter 1. beschriebenen Antikörperstandardlösungen aufgestellt werden kann.

Das nachstehende Beispiel dient zur näheren Erläuterung der Erfindung:

Beispiel

1. Isolierung einer niedermolekularen Glycopeptidfraktion (Zuckerderivat der Formel I)

1.1. Isolierung der Zellwand von Bacillus subtilis var. aterrimus

Bacillus subtilis wurde in Massenkulturen (40 l) gezüchtet, die Zellen in der spätlogarithmischen Wachstumsphase geerntet und mit Trypsin behandelte Zellwände nach der Methode von Schleifer und Kandler (Schleifer, K.H. und Kandler, O. (1972): Peptidoglycan types of bacterial cell walls and their taxonomic implications. Bact. Rev. 36, 407 - 477) isoliert.

1.2. Extraktion von Proteinen mittels Subtilisin

Mit Trypsin behandelte Zellwand wurde nach dreistündiger Extraktion bei 60°C mit 10%iger Trichloressigsäure (5 mg Zellwand/ml) mit der Protease Subtilisin weiter gereinigt.
Mit Trichloressigsäure extrahierte Zellwand (2 mg/ml) wurde in 0,1 M Phosphatpuffer (pH = 7,5) suspendiert und über Nacht mit Subtilisin (11 Units/mg; BPN'EC 3.4.21.14) bei 37°C inkubiert. Nach Zentrifugieren wurden die Zellwände viermal gewaschen und lyophilisiert.

1.3. Hydrolyse der Zellwand mit Lysozym

Subtilisin-behandelte Zellwand wurde in 0,1 M Ammoniumacetatpuffer (pH = 6,2) suspendiert, mit 10 mg Lysozym/ml (Serva: 22000 Units/mg) versetzt und für ca. 24 Stunden bei 37°C inkubiert. Zur Verhinderung von Bakterien- und Pilzwachstum wurden einige Tropfen Toluol zugesetzt.

1.4. Isolierung des niedermolekularen Glycopeptids (Zuckerderivat der Formel I)

Die Lysozym-behandelte Zellwandsuspension wurde bei 48000 g 20 Minuten zentrifugiert, der Überstand zweimal 4 Stunden gegen $H_2O$ bidest. dialysiert. Das Dialysat konzentrierte man im Vakuum bei 40°C und lyophilisierte es anschließend.

1.5. Gelchromatographie des Dialysats an Sephadex

Der ölige Rückstand wurde über $P_2O_5$ getrocknet und in $H_2O$ bidest. aufgenommen (50 mg/0,1 ml). Nicht lösliche Bestandteile wurden durch Zentrifugation abgetrennt. Fraktionen zu 100 µl wurden an Sephadex G 25 coarse chromatographiert. Die Abmessung des Säulenbetts betrug 100 x 2,2 cm. Die Elution erfolgte mit $H_2O$ bidest. bei einer Geschwindigkeit von 4 Tropfen/Minute. Es wurden Fraktionen zu 2 ml gesammelt, ihre Absorption bei 206 nm bestimmt, die glycopeptidhaltigen Fraktionen vereinigt und gefriergetrocknet.

2. Herstellung des Konjugats Human-Serum-Albumin-Glycopeptid

15 mg Humanserumalbumin wurden mit 46,2 mg Glycopeptid in 2,85 ml Carbonatpuffer (0,1 M, pH 9,6) gelöst. Die Lösung wurde mit 150 µl 25%igem Glutaraldehyd (Sigma) versetzt und über Nacht bei 4°C gerührt. Zur Absättigung noch freier Aldehydgruppen wurde das Reaktionsgemisch 3 Stunden mit L-Lysin (0,2 mol) inkubiert, nach erschöpfender Dialyse gegen $H_2O$ bidest. dialysiert und der Überstand lyophilisiert.

3. Adsorption des Konjugats an Kunststoffoberflächen, z.B. Polystyrol

Im vorliegenden Beispiel wurden Polystyrolröhrchen mit einem Fassungsvermögen von knapp 2 ml, 4 cm hoch, Durchmesser: 1 cm, verwandt. Zur Bindung eignen sich auch z.B. Mikrotiterplatten, Kugeln, Küvetten etc. aus gleichartigem Kunststoffmaterial.

50 µl Konjugatlösung (entsprechend 100 ng Konjugat in 0.1 molarem Bicarbonatpuffer pH 9.6) werden in die Röhrchen einpipettiert und über 24 Stunden bei 37°C getrocknet. Die trockenen Röhrchen werden jeweils einmal mit 200 Mikroliter 10 mmol/l Phosphatpuffer pH 7.2 in isotoner Kochsalzlösung enthaltend 0.1 % PBS (phosphate buffered saline)/Tween 20 gewaschen und nach Absaugen der Waschlösung nochmals mit 200 Mikroliter destilliertem Wasser gespült und wieder abgesaugt. Die mit dem Konjugat beschichteten Röhrchen werden im Trockenschrank bei 37°C aufbewahrt.

## 4. Durchführung des Testes

100 Mikroliter spezifische Standardlösung, enthaltend 0.1 µg bis 10 µg IgG (siehe Standardherstellung) in PBS/Tween 20-Puffer, werden in die wie oben beschriebenen beschichteten Röhrchen einpipettiert und 1 Stunde bei 20°C inkubiert. Untersuchungen von biologischem Material, z.B. Patientenseren, werden, um in den Meßbereich der Standardkurve zu gelangen, je nach Titer 1:5 bis 1:2000 mit PBS/Tween 20-Puffer verdünnt und in gleichem Volumen eingesetzt. Der Inkubationsansatz wird abgesaugt und anschließend zweimal mit 200 Mikroliter PBS/Tween 20-Puffer gewaschen und wieder abgesaugt. 100 µl (Antihuman IgG)-Immunglobulin (z.B. von der Ziege) markiert mit Peroxidase oder alkalischer Phosphatase werden in die Röhrchen einpipettiert und 1 Stunde bei konstanter Temperatur entweder bei 20°C, 25°C oder 37°C inkubiert. Anschließend wird nochmals zweimal mit je 20 Mikroliter PBS/Tween 20-Puffer gewaschen. Bei Verwendung des Indikatorsystems konjugierte Peroxidase werden als Substrat o-Phenylendiamin und $H_2O_2$ in Citratpuffer 0,1 mol/l, pH 5.0, zugesetzt und 15 Minuten bei konstanter Temperatur inkubiert.

Zum Stoppen der Reaktion werden 50 µl 5 normale $H_2SO_4$ zugesetzt. Die Extinktion bei 492 nm ist proportional zur eingesetzten Immunglobulinmenge. In Humanseren findet man Konzentrationen von 1 µg pro ml bis in den mg-Bereich.

Der Variationskoeffizient für die Bestimmung der spezifischen IgG's liegt im mittleren Bereich der Eichkurve bei 6.3 %.

0150466

## Gewinnung eines spezifischen humanen Immunglobulin-G-Standards

1. Kopplung des Albumin-Glycopeptid-Konjugats an Sepharose 4 B

10 ml Sepharosegel werden fünfmal mit 4- bis 5-fachem Überschuß an destilliertem Wasser gewaschen und dann in 10 ml Wasser suspendiert. Dazu werden 430 mg Bromcyan, gelöst in 8 ml Wasser, gegeben. Die Reaktion findet bei Raumtemperatur statt, der pH-Wert wird auf 11.0 durch Zugabe von 2 mol/l Natronlauge konstant gehalten. Nach 15-minütiger Reaktion unter leichtem Rühren wird das Gel auf einem Büchnertrichter abgesaugt und mit 60 ml 0.1 mol/l Natriumhydrogencarbonatlösung nachgewaschen. Die Sepharose wird in 7.5 ml 0.1 mol/l Natriumhydrogencarbonat, die 50 mg Albumin-Glycopeptid-Konjugat enthalten, eingerührt. Die Kopplung erfolgt über Nacht bei 4°C unter leichtem Rühren. Nach der Reaktionszeit wird wieder auf der Filternutsche abgesaugt und das gekoppelte Gel jeweils zweimal mit 30 bis 40 ml Wasser, dann 0.1 mol/l Natriumhydrogencarbonat, 0.2 mol/l Natriumacetatpuffer pH 4.5, 0.2 mol/l Natriumphosphatpuffer pH 7.2 und 0,01 mol/l PBS 7.2 gewaschen. Das Affinitätsgel wird in PBS-Puffer pH 7.2 bei 4°C aufgehoben.

2. Durchführung der Affinitätschromatographie

Das Gel wird in eine passende Säule gefüllt und antikörperhaltiges Serum beziehungsweise eine angereicherte Immunglobulinfraktion aufgegeben. Die beschickte Säule wird mit PBS-Puffer (ohne Tween) gewaschen bis die Extinktion bei 280 nm im Eluat unter 0.03 absinkt (Verbrauch ca. die doppelte Menge Puffer wie Serum).
Die Elution erfolgt mit Essigsäure (0.1 - 1 mol/l). Der Antikörper wird mit den ersten Fraktionen der Essigsäure eluiert, die antikörperhaltigen Fraktionen werden vereinigt und neutralisiert. Die vereinigten Eluate werden gegen eine ausreichende Menge PBS dialysiert, konzentriert und anschließend auf Sephadex G 200 chromatographiert.

3. Charakterisierung der Antikörperfraktion

Das gereinigte Immunglobulin wurde als symmetrischer Peak, vermessen bei 280 nm, von einer geeichten Sephadex-G-200-Säule eluiert. Das Molekulargewicht dieser Fraktion lag bei etwa 150.000. Eine weitere Absicherung des Molekulargewichts und der Homogenität erfolgt durch analytische Ultrazentrifugation, vermessen mit der UV-Optik bei 280 nm. Das Protein sedimentierte als einheitliche Bande. Unter Standardbedingungen ergab sich eine $S_{20w}$ von 7.8. Auf der Celluloseacetatelektrophorese ergab sich eine einheitliche Bande im Bereich der Gammaglobuline.
In der Immunelektrophorese war bei Verwendung eines polyvalenten Anti-Serums eine Präzipitationslinie im Immunglobulin-G-Bereich nachzuweisen.

0150466

Patentansprüche

1. Antikörper aus der Klasse der Immunglobuline G, A und M in biologischem Untersuchungsmaterial gegen den Glycanstrang des Peptidoglycans, gekennzeichnet durch

a) spezifische Bindung an Zuckerderivate der allgemeinen Formel

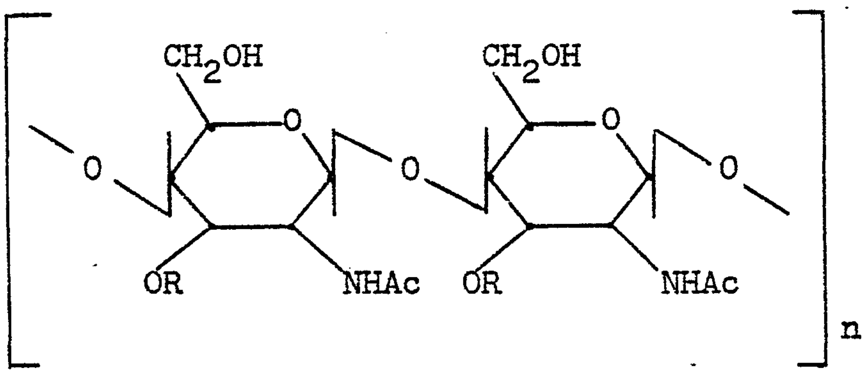

(I)

worin

R Wasserstoff oder einen der Reste

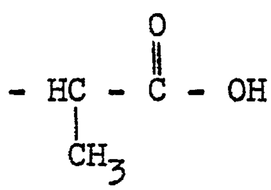

oder

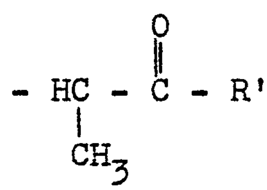

,

R' den Rest einer Aminosäure oder eines Peptids und

n eine der Zahlen 1 bis 6, vorzugsweise 2 - 4 bedeuten, oder

b) spezifische Bindung an Zuckerderivate der allgemeinen Formel I enthaltende isolierte bakterielle Zellwände.

2. Antikörper nach Anspruch 1, dadurch gekennzeichnet, daß in Formel I der Rest R Wasserstoff oder einen der Reste

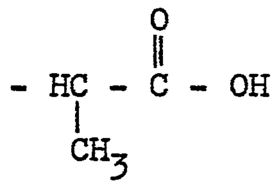 oder 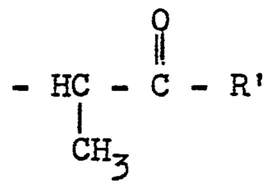 ,

R' eine der Aminosäuren L-Ala, L-Ser, Gly; bzw. die Peptide
L-Ala-D-Glu;
L-Ala-D-i-Gln;
L-Ala-D-Glu(L-Lys);
L-Ala-D-Glu($A_2$pm) und
n eine der Zahlen 2 bis 4 bedeuten.

3. Verfahren und Reagenzien zur Herstellung der Antikörper gegen den Glycanstrang des Peptidoglycans sowie gegen diesen Glycanstrang enthaltende bakterielle Zellwände, dadurch gekennzeichnet, daß ein Zuckerderivat der allgemeinen Formel I gemäß Anspruch 1 nach an sich bekannten Methoden an eine feste Phase gekoppelt und in dieser Form zur selektiven Bindung und anschließender selektiven Elution des spezifischen Antikörpers aus der Klasse der Immunglobuline G, A und M aus einer durch Fällungsreaktion angereicherten Immunglobulinfraktion aus menschlichem Plasma verwendet wird.

4. Verfahren und Reagenzien zur quantitativen Bestimmung von Antikörpern aus der Klasse der Immunglobuline G, A und M gegen den Glycanstrang des Peptidoglycans in biologischem Untersuchungsmaterial nach an sich bekannten immunochemischen Verfahren, dadurch gekennzeichnet, daß man als Antigen eine Verbindung der allgemeinen Formel

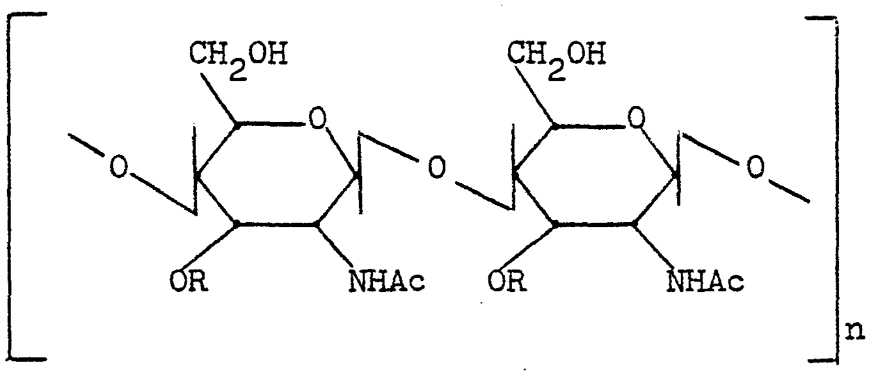

(I)

worin

R Wasserstoff oder einen der Reste

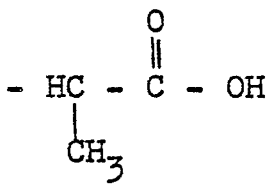

oder

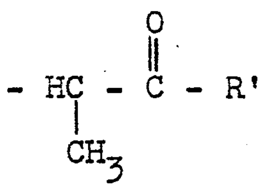

,

R' den Rest einer Aminosäure oder eines Peptids und

n eine der Zahlen 1 bis 6 bedeutet, verwendet.

5. Verfahren nach Anspruch 4, dadurch gekennzeichnet, daß die quantitative Bestimmung von Antikörpern in einem Doppel-Antikörper-Enzymimmun-Assay durchgeführt wird.

6. Verfahren nach Anspruch 4, dadurch gekennzeichnet, daß die quantitative Bestimmung von Antikörpern mittels einer nephelometrischen Methode durchgeführt wird.

7. Verfahren nach Anspruch 4, dadurch gekennzeichnet, daß die quantitative Bestimmung von Antikörpern in einem Doppel-Antikörper-Radioimmun-Assay durchgeführt wird.

8. Verfahren nach Anspruch 4, dadurch gekennzeichnet, daß die quantitative Bestimmung von Antikörpern in einem Festphasen-Sandwich-Radioimmun-Assay durchgeführt wird.

9. Verfahren nach Anspruch 4, dadurch gekennzeichnet, daß die quantitative Bestimmung von Antikörpern in einem Festphasen-Enzymimmun-Assay durchgeführt wird.

10. Verfahren nach Anspruch 4, dadurch gekennzeichnet, daß die quantitative Bestimmung von Antikörpern in einem Festphasen-Fluoreszenz-Immun-Assay durchgeführt wird.

11. Verfahren nach Anspruch 4, dadurch gekennzeichnet, daß die quantitative Bestimmung von Antikörpern mittels eines Latex-Agglutinationstests durchgeführt wird.

12. Verwendung des nach Anspruch 3 hergestellten spezifischen Immunglobulins der Klasse G, A und M als Standard zur quantitativen Messung oder als Positivkontrolle für die in den Ansprüchen 4 - 11 aufgeführten Verfahren.